Europäisches Patentamt

European Patent Office

Office européen des brevets

(11) Veröffentlichungsnummer: **0 350 517 B1**

(12) **EUROPÄISCHE PATENTSCHRIFT**

(45) Veröffentlichungstag der Patentschrift: **22.07.92**

(21) Anmeldenummer: **88111098.5**

(22) Anmeldetag: **12.07.88**

(51) Int. Cl.⁵: **A43B 7/14**

(54) **Medizinisch orientierte Einlage für Schuhe, insb. Sportschuhe.**

(43) Veröffentlichungstag der Anmeldung:
**17.01.90 Patentblatt 90/03**

(45) Bekanntmachung des Hinweises auf die
Patenterteilung:
**22.07.92 Patentblatt 92/30**

(84) Benannte Vertragsstaaten:
**AT BE CH DE ES FR GB GR IT LI LU NL SE**

(56) Entgegenhaltungen:
EP-A- 0 205 125     DE-A- 1 685 782
DE-C- 849 812      DE-U- 1 636 111
DE-U- 1 928 411     US-A- 1 386 531
US-A- 2 581 864

(73) Patentinhaber: **KLEYLEIN, Horst**
**Sudetenstrasse 13**
**W-8502 Zirndorf(DE)**

(72) Erfinder: **KLEYLEIN, Horst**
**Sudetenstrasse 13**
**W-8502 Zirndorf(DE)**

(74) Vertreter: **Merten, Fritz**
**Tristanstrasse 5**
**W-8500 Nürnberg 40(DE)**

Rank Xerox (UK) Business Services

# Beschreibung

Die Erfindung betrifft eine medizinisch orientierte Einlage für Schuhe, insb. Sportschuhe, wie Tennisschuhe, Basketballschuhe u. a. mit über dem Knöchel des Fußes reichendem Schaft und mit seitlich des Knöchels anlegbaren und diesen mindestens teilweise umgreifenden Polstern, wobei die Polster jeder Seite des Knöchels von zwei L-förmig zueinander stehenden Schenkeln gebildet und der Knöchel durch diese einerseits vertikal und andererseits rückwärtig abstützbar ist.

Aus vielen Untersuchungen von Bewegungsabläufen des Menschen, so beispielsweise Gehen, Laufen, Springen u. a., ist seit langem bekannt, daß dem jeweiligen Fuß eine enorme Belastung zugemutet wird, die dieser meist durch entsprechendes Einstellen zur Lauffläche einerseits und Abrollen dessen Fußteiles andererseits bewältigt. Die Konzeption des Fußes als solche hat sich diesen Bewegungsabläufen weitgehend angepaßt, und es treten relativ wenige Fußschäden auf, wenn der Fuß, ohne Schuhwerk, diesen Bewegungsabläufen, insb. auf natürlichem, weichem Boden, ausgesetzt wird. In der heutigen, zivilisierten Gesellschaft mit beschuhten Bewegungsabläufen gilt es, dem Fuß Hilfsmittel zur Verfügung zu stellen, mit denen dieser den ihm eigentümlichen Bewegungsabläufen folgen kann, ohne dabei selbst Schaden zu nehmen, bzw. den zu tragenden Körper zu schädigen.

Aus den vorstehenden Untersuchungen ist auch bekannt, daß beim Auftreten des Menschen über direkten Druckkontakt am Fersenbein bzw. an den hier wirkenden Sehnenansätzen Kräfte frei werden, die dem 6-fachen des Körpergewichtes entsprechen können. Am Fuß und besonders auch an der Fußsohle wird bekanntlich das dynamische Bewegungsmoment des, z. B. sich vorwärts bewegenden Körpers, in einen statischen Zustand umgewandelt, d. h. die Fußsohle sitzt auf dem Boden auf bzw. rollt ab, wodurch Scherkräfte, insb. am Fersenbein als Folge dieses Bewegungsablaufes entstehen. Auch haben solche Untersuchungen ergeben, daß der Fuß und hier insb. der Rückfuß bei diesen Bewegungsabläufen diversen Querverformungen ausgesetzt wird. Diese Querverformungen führen dazu, daß das Fersenbein beim Laufen in einer O-Stellung (Valgus) auf den Boden aufgesetzt und von diesem wieder abgehoben wird, daß es aber in der Abrollphase eine Knickung in der X-Stellung (Varus) durchläuft und folglich auch in dieser Bewegung abgebremst und beschleunigt werden muß.

Demzufolge treten, z. B. in der Laufbewegung, im Rückfuß diverse Probleme im Abfangen der Belastungsabläufe des Fußes auf, die statisch-mechanischen bzw. statisch-dynamischen Ursprungs sind.

Die Dämpfungsfunktion beim Auftreten, bei der das Hüftgelenk, insb. beim Laufen, keine Erschütterung mehr erfährt, kann von einem weichen Boden, einem weichen Schuh oder von den Sehnen und Muskeln im Bereich des Fußes und Unterschenkels übernommen werden.

Werden solche Bewegungsabläufe hingegen ruckartig eingesetzt oder halten diese chronisch länger an, so überlasten sie die Muskelzüge, wie z. B. die Achillessehne oder die Plantarfascie und führen zu anhaltenden Beschwerden, wie Fersenspornbeschwerden, Achillodynien u. a.

Aus vielen Beobachtungen und der Erfahrung heraus führen die Bewegungsabläufe mehr und mehr zu anhaltenden Schädigungen der Füße, zumal diese in modisch orientierte und weniger in orthopädisch geformte Schuhe gezwengt werden und die Bewegungsabläufe meist auf harten Böden stattfinden, die weder eine notwendige Dämpfung des Fußes, noch einen natürlichen Abrollvorgang ermöglichen.

Um diesen Problemen in einer oder anderer Weise entgegenzuwirken, ist eine Vielfalt orthopädischer Schuhe und/oder Einlagen für diese entwickelt worden, von denen die meisten, sich Teillösungen mit mehr oder weniger großem Erfolg widmen.

So ist beispielsweise durch die DE-OS 23 38 060 ein orthopädisch, therapeutischer Schuh bekannt geworden, bei dem auf einer im Bezug auf den Träger nach außen weisenden Seite des Schuhs zwischen Innenfutter und Schaft im Bereich des Knöchels Scheiben vorgesehen sind, die im wesentlichen schräg nach abwärts und senkrecht abwärts verlaufen und in die Stützstäbe einschiebbar sind. Die im eingeschobenen Zustand zum Teil vor und zum Teil hinter dem Knöchel am Fersenbereich anliegenden Stützstäbe versteifen den Bereich des Knöchels am Schaft des Schuhs und bilden dort eine den Knöchel umspannende Versteifung in Form eines auf dem Kopf stehenden "V". Derartige Stützstäbe mögen zwar eine verstärkte Abstützung und dadurch einen zusätzlichen Halt zu geben, sie behindern jedoch erheblich den natürlichen Bewegungsablauf, insb. den Fußabrollvorgang beim natürlichen Gehen.

Auch versteifen diese Stützstäbe den Schaft des Schuhs so stark, daß selbst in diesem Bereich Druckstellen entstehen, die nachteilig auf den Knöchel und damit auch auf das Fußgelenk wirken. Auch hat man festgestellt, daß eine Abstützung des Knöchels nicht von oben, Gabel des "V", sondern von unten kommen müsse, wenn die stützende Funktion des Knöchels wirksam werden soll.

Um diese stützende Funktion des Knöchels von unten vorzunehmen, ist durch die CH-PS 337 751 eine Einlage für Sportschuhe bekanntgeworden, die aus zwei L-förmigen Schenkeln an jeder Seite des Knöchels besteht, in deren Verbindungsebene der Knöchel eingebettet ist. Beide Hälften der Einlage sind als aufblasbare Teile ausgeführt, und es sind die Schenkel dieser Einlage derart zueinander angeordnet, daß der eine Schenkel seitlich unterhalb des Knöchels, und der andere Schenkel im wesentlichen senkrecht und parallel zur Achillessehne verläuft. Die Lufträume der senkrecht verlaufenden Schenkel beider Knöchelseiten sind entlang der Achillessehne miteinander verbunden, und es sind diese Lufträume mit den Lufträumen, die unterhalb des Knöchels liegen, verbunden. Parallel zur Achillessehne verläuft auch ein Ventil, durch das die aufblasbaren Polster mit Luft gefüllt werden können.

Zwar lassen sich mit einer solchen Einlage Hohlräume zwischen dem Fußgelenk und der Innenseite des Schaftes des Schuhs ausfüllen, um einen besseren Kontakt zwischen diesen herzustellen, indessen wird es hier als nachteilig angesehen, daß hier weder auf die unterschiedliche Achsenlage des Knöchels, in Bezug auf dessen Stützung, Rücksicht genommen, noch der Bewegungsablauf des Fußes im Schuh dämpfend, unterstützt wird. Aus diesem Grund ist diese Einlage auch nicht geeignet, dem Fuß die erforderliche Führung und Stützung, und insb. Dämpfung, gegenüber dem Fußbett zu verleihen.

Eine Einlage für ein Sportschuhwerk ist auch aus der DE-U-1 928 411 bekannt.

Ferner ist durch die DE-AS 1 015 364 eine Einlage für den Schutz des Knöchels und Fußgelenkes bekannt, die aus je einem beidseitig des Knöchels anbringbaren, U-förmigen Polsterwulst besteht, in dessen gerundete, tiefste Stelle der Knöchel eingebettet werden kann. Die Schenkel dieses Polsterwulstes sind senkrecht oder annähernd senkrecht nach oben gerichtet, und es wird mit diesem der Lage der Gelenkachse insofern Rechnung getragen, als die Polsterwülste der beiden Knöchelseiten mit deren Schenkeln unterschiedlich angestellt sind. Hierbei verlaufen die hinter dem Knöchel liegenden Schenkel zum einen nahe der Achillessehne und zum anderen abgesetzt von diesser, und es sind die Schenkel selbst unterschiedlich lang ausgeführt. Die Polsterwülste selbst sind an einem Fußling aus einem gummielastischen Gewirke angebracht, welches vom Schaft des Schuhs bis zum Gewölbe des Fußes reicht, und bestehen aus einem hochelastischen Schaumstoff, der einen bohnenförmigen Querschnitt aufweist. Mit dieser Einlage wird zwar die unterschiedliche Lage der Knöchel berücksichtigt, es wird aber nicht erkannt, daß neben der Stützung des Knöchels auch das Fußgelenk selbst und der Bereich des Fußgewölbes, insb. an der Plantaraponenrose, dämpfend abgestützt und der Fuß sowohl in Längsrichtung als auch quer zu dieser geführt werden muß.

Durch die DE-PS 855 370 ist ein orthopädischer Schuh bekannt, bei dem zur nachgebenden Abstützung der Ferse des Fußes zwischen der Brandsohle des Schuhes und dessen Absatzes eine Lochaussparung vorgesehen ist. Die Lochaussparung, die bis auf einen Rand die überwiegende Fläche des Absatzes umspannt, wird durch Keileinlagen gebildet, die zwischen Brandsohle und Absatz eingelegt sind. Zwar wird hier eine gewisse Nachgiebigkeit der Brandsohle im Bereich der Ferse erzielt, diese ist aber für eine Einbettung erkrankter Teile der Ferse, z. B. des Fersensporns, von untergeordneter Bedeutung, weil die rundum am Schuh eingespannte Brandsohle, infolge ihrer randseitigen, festen Fixierung und Härte, kaum nachgibt und somit beispielsweise der Fersensporn kaum in die Lochaussparung eingebettet wird. Auch kann dieser Schuh dem natürlichen Bewegungsablauf des Fußes nicht folgen, und trägt in keiner Weise bei, den Knöchel abzustützen und/oder das Fußgewölbe zu führen.

Um dem Fuß im Bereich des Fußgewölbes eine Führung und Abstützung zu geben, ist gem. der DE-PS 900 179 bekannt, eine Einlage in den Schuh einzubringen, die von zwei sandwichartigen Zwischensohlen und einer balgförmigen Erhöhung, d. h. Gelenkeinlage, besteht. Diese Gelenkeinlage, deren Erhöhung im wesentlichen aus einer Materialanhäufung besteht, ist dreieckförmig ausgeführt und so zwischen den Zwischensohlen eingelegt, daß sie mit einer Schenkelseite quer zur Längsachse des Schuhs steht und dem Fußballen dort ihre höchste Erhebung bietet. Mit dieser Einlage wird der Bewegungsvorgang in Längsrichtung des Schuhs dämpfend gebremst, doch kann diese Einlage keine weiteren Funktionen in Bezug auf den Bewegungsablauf übernehmen, so daß auch hier nur eine Einlage vorliegt, die einer ganz engen Teilfunktion gerecht wird.

Ausgehend von diesen, entgegen zu wirkenden Problemen und der Erkenntnis, daß der Fuß beim Abrollen auf dem Boden eine spezielle Bewegungsfolge durchläuft, die wie folgt beschrieben werden kann:

- Aufsetzen des Rückfußes in O-Beinstellung, des Fersenbeines mit ausgeprägtem Längsgewölbe im Mittelfußbereich,
- Abrollen in X-Beinstellung des Rückfußes mit abgeflachtem Längsgewölbe,
- in der Absatz- und Endschubphase Belastung des Vorderfußes; dabei erneutes Ausformen des Längsgewölbes, so wie
- leichtes Drehen im Bereich des Mittelfußgelenkes sowie Rückkehr in die O-Beinstellung des Rückfußes,

liegt der Erfindung die Aufgabe zugrunde, eine Einlage, für insb. einen Sportschuh, dahingehend weiterzubilden, daß sowohl die im Bänderverbund zwischen Knöchel und Rückfuß (Sprung- und Fersenbein) auftretenden Zugkräfte von der Einlage aufgenommen und in den Schuh übertragen werden, als auch der normale Bewegungsablauf des Fußes, wie oben ausgeführt, nicht, zumindest aber nicht wesentlich, beeinträchtigt wird.

Diese Aufgabe wird durch eine Einlage gemäß dem Anspruch 1 gelöst.

Durch diese Maßnahmen wird nicht nur die der Erfindung zugrunde liegende Aufgabe vorteilhaft gelöst, sondern es werden die weiteren, nachfolgenden Vorteile erzielt. So ist diese Einlage einmal aufgrund ihrer besonderen Form in Bezug auf bodenlagige und seitliche Stützung des Fußes und zum anderen aufgrund ihrer Materialzusammensetzung in der Lage, die vom Boden und/oder vom Schuh nicht mehr gegebene Dämpferfunktion zu übernehmen und damit entlastend auf die knöchernen, die bandmäßigen als auch muskularen Strukturen im Fuß- und Unterschenkelbereich positiv einzuwirken.

Ein weiterer Vorteil besteht darin, daß durch die Verdickung bzw. Materialanhäufung am vorderen Innenrand der Fußbettstütze der sogenannte Peitschenschlag-Mechanismus der Verspannung an der Fußsohle gefedert wird. Dies führt zu einer zeitweiligen Richtungsänderung der unter Zug stehenden Strukturen des Fußes und damit zur Vermeidung lokaler Zugspitzen im Bereich der Ansatzstelle am Fersenbein und somit auch zur Vermeidung der sogenannten Fersensporbeschwerden (Knochenhautentzündung, Periostose, Insertionstendinose).

Daneben entsteht durch die federnde Aussparung im Bereich der Fußsohle eine Druckentlastung an dieser Ansatzstelle, gleichzeitig aber auch ein Massageeffekt, der sowohl antiödematös (entschwellend) als auch durchblutungsfördernd wirkt und in diesem Zusammenhang sowohl prophylaktische als auch therapeutische Effekte entwickelt.

Druck- und Zugbelastungen entstehen auch durch die Umsetzung der dynamischen Bewegung des Körpers in ein statisches Element an der Fußsohle, wodurch der Fuß im Schuh nach vorne rutscht. Auch hier wirkt die mediale, vordere Wulstung im Sinne einer Bremse sowie einer großflächigen und günstigeren Druckaufnahme im Sinne eines Hemmschuhs den Fersenbeinperiostosen entgegen.

Überlastungsbeschwerden im unteren Sprunggelenk bzw. der dieses Gelenk übergreifenden Muskel- und Sehnenstrukturen treten sowohl im Rahmen des Abrollvorganges und der dabei erforderlichen Wechselstellung, z. B. X-O-X auf, als auch durch Ermüdung bei längerem Stehen durch die dann bestehende X-Bein-Stellung des Rückfußes.

Im Stand entlastet die Einlage bzw. Orthese die statisch aktiven Strukturen, hingegen wird beim Laufen bzw. in der Gehphase, entsprechend der im Fußaußen- und Innenrand stehenden Aufkeilung, beim Auftreten die richtige Fersenbeineinstellung induziert.

Bei Problemfüßen, beim X-Bein, beim O-Bein, beim X-Rückfuß, beim O-Rückfuß können an die Orthese zusätzliche Aufkeilungen an deren Sohle (sogenannte Innen- oder Außenranderhöhungen) aufgebracht werden, die dann eine nicht ideal stehende Fersenbeineinstellung korrigieren. Auch helfen diese, dann Überlastungsschäden, insb. beim Sportler, an der Achillessehne sowie im Bereich des Längsgewölbes zu vermeiden.

Bei Außenbandschäden des oberen Sprunggelenkes - statistisch gesehen, die am häufigsten verletzte, isolierte Struktur beim Menschen - kann eine Außenranderhöhung evtl. sogar in Kombination mit einer pelottenförmigen Erweiterung der Orthese - eine Stabilisierung bzw. Entlastung dieser Problemstrukturen erzielen.

Damit ist diese Einlage bzw. Orthese hervorragend geeignet, zur Vermeidung statischer Beschwerden im Alltag, ferner zur Vermeidung von Überlastungsbeschwerden bei stehenden Berufen sowie im Freizeitsport, eingesetzt zu werden. Auch ist diese Einlage sehr gut geeignet, bei Leistungssportlern diversen Sportschäden bzw. Verletzungen entgegenzuwirken.

Weitere Vorteile bietet diese Einlage bzw. Orthese bei der Behandlung des sogenannten Problemfußes mit Fehlstellung im Rückfußbereich. Auch in der Nachbehandlung von konservativ oder operativ zu behandelnden Überlastungsschäden bzw. Band- oder Sehnenrupturen und hier insb. an der Achillessehne und Außenbandapparat am oberen Sprunggelenk ergeben sich neue Perspektiven einer erfolgreichen Behandlung.

Eine vorteilhafte Weiterbildung dieser Einlage zeichnet sich dadurch aus, daß die Pelotten an ihren, der Fußbettstütze zugewandten Seite, einen Saum aufweisen, und daß diese über diese Säume sowohl miteinander als auch mit dem Schuh verbunden sind, und daß die Fußbettstütze über diese Säume auf dem Fußbett aufgelegt ist.

Diese Verbindung der Pelotten unter der Ferse hat den Vorteil, daß die Verbindungsteile dort leichter untergebracht und zudem keine Druckstellen an der Ferse entstehen, die zu neuen Beschwerden Anlaß geben könnten. Auch hat diese Verbindung den Vorteil, daß die den Knöchel stützenden Polster an den Pelotten ihre Lage besser und vor allem genauer halten können, so daß der Knöchel, die Ferse und der Fuß selbst exakt auf dem Fußbett aufgelegt werden können.

Ein weiteres Merkmal der Erfindung besteht darin, die Polster und/oder die Fußbettstütze aus einem elastischen Werkstoff (Siliconkautschuk oder Schaumstoff) auszuführen.

Dies hat den Vorteil, daß im Gegensatz zu harten Polsterungen für den Knöchel, die Ferse u. a., hier eine weiche, dämpfende und hautsympathische Polsterung bzw. Stütze geschaffen wird, die dem einwirkenden Druck ausreichend, nachgebend folgt und dennoch die notwendige Führung und Abstützung des Fußes im Schuh gewährleistet. Ein weiterer Vorteil dieses Materials liegt auch darin, daß selbst nach Verformungen desselben durch die Stütz- und/oder Druckeinwirkung dieses in seine Ausgangslage wieder zurückgeht und somit die gewollte Funktion, selbst nach längerem Gebrauch, sicherstellt.

Eine weitere, vorteilhafte Ausführung dieser Einlage besteht darin, daß deren Teile, bestehend aus mindestens den pelottenförmigen Polstern für den Knöchel und/oder der Fußbettstütze für die Auflage des Fußes als eine, am Schuh integrierte, feste Einheit ausgeführt und diese somit Bestandteil des Schuhs ist.

Diese Variante hat den Vorteil, daß schon ab Fabrik Schuhe hergestellt werden können, die den orthopädischen Ansprüchen weitgehend gerecht werden. Die allgemeine Anpassung der Schuhe dieser Art kann gegebenenfalls einige, möglichst engere Variationen beinhalten, so daß den meisten Menschen mit unterschiedlich großen bzw. geformten Füßen geholfen werden kann. Diese Variante hat den Vorteil, daß auch dem modischen Aspekt Rechnung getragen werden kann, da schon bei der Fertigung des Schuhs die Raumverhältnisse für die Unterbringung der Einlage berücksichtigt werden können.

Gemäß einer weiteren, vorteilhaften Ausbildung der Einlage können bei dieser mindestens die pelottenförmigen Polster für den Knöchel und die sie haltenden Scheiben in einem formelastischen Gewirk eingestrickt sein, und sie können zusammen eine Einheit bilden, die in einen dafür passenden Schuh einsetzbar sind.

Durch diese Maßnahmen wird der Anwendungsbereich der Einlage noch weiter ausgedehnt, da neben sogenannten orthopädischen Schuhen auch annähernd beliebig andere verwendet werden können. Auch kann eine solche, an einem Gewirk hängende Einlage in einer Rehabilitationsphase mit wechselndem Schuhwerk verwendet werden, wodurch der Fuß entsprechend geführt und gestützt wird, das Schuhwerk hingegen, je nach Anwendungsfall wechseln kann.

In den Zeichnungen ist eine Ausführungsform der Einlage, deren Konzeption und Teile am Beispiel eines Sportschuhs mit Schaft schematisch dargestellt. Es zeigt:

Fig. 1 eine perspektifische Ansicht auf einen Sportschuh mit der am fersenseitigen Ende des Schuhs gestrichelt dargestellten Einlage,

Fig. 2 eine perspektifische Ansicht auf die Einlage mit den den Knöchel abstützenden Pelotten und einer von der Ferse bis zum Fußballen reichenden Fußbettstütze,

Fig. 3 einen Längsschnitt durch den Schaft des Sportschuhs im Bereich der Achillessehne und die Einlage mit deren den Knöchel abstützenden Polstern und die Pelotte im Schnitt,

Fig. 4 eine Seitenansicht einer nur den Bereich der Ferse erfassenden Fußbettstütze mit je einer, am Fußaußenrand und -innenrand stehenden Aufkeilung,

Fig. 5 eine Draufsicht auf die Fußbettstütze gemäß Fig. 4,

Fig. 6 eine Draufsicht auf die Fußbettstütze gemäß Fig. 4, jedoch betrachtend von deren Auflage auf der Brandsohle, mit einer eiförmigen Ausformung für die Aufnahme, z. B. des Fersensporns,

Fig. 7 einen Schnitt durch die Fußbettstütze in der Ebene VII - VII in Fig. 6,

Fig. 8 eine Draufsicht auf eine Fußbettstütze, die von der Ferse bis zu den Zehen reicht und mit einer, von deren äußeren Randzone zum Fußballen sich bogenförmig erstreckenden, Aufpolsterung,

Fig. 9 ein L-förmiges, den inneren Knöchel stützendes Polster und die nur angedeutete, dieses Polster haltende Scheibe bzw. Pelotte,

Fig. 10 einen Schnitt durch das Polster und dessen Scheibe in der Ebene VIII - VIII gem. Fig. 9,

Fig. 11 eine Form für das Abgießen der elastischen Polster und hier für die Innenseite des Knöchels, und

Fig. 12 einen Schnitt durch die Form in der Ebene XII-XII in Fig. 11

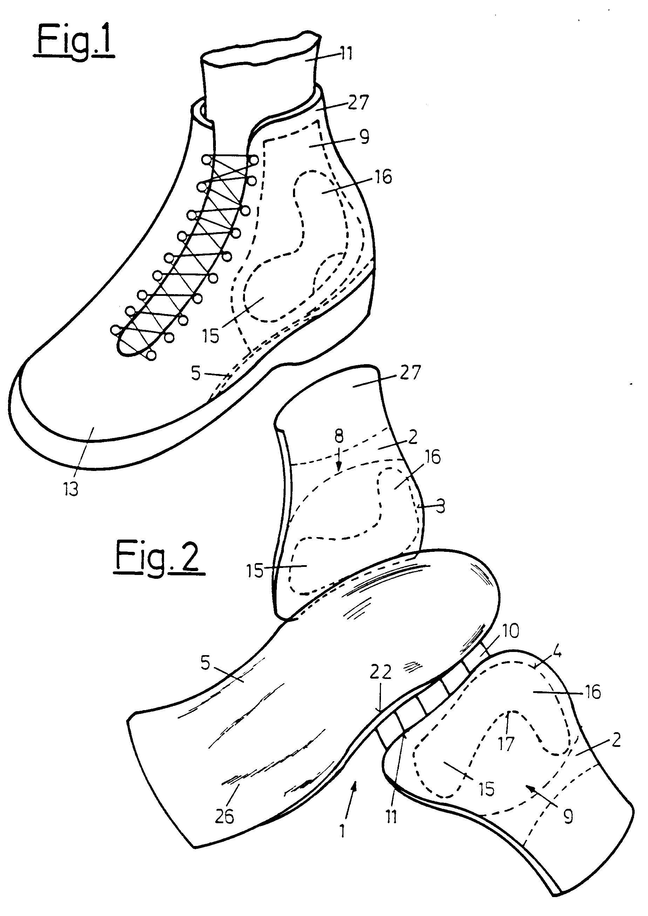

Die Einlage 1 gem. dem Ausführungsbeispiel nach den Fig. 1 - 3 wird im wesentlichen von einer Pelotte 2 mit zwei in dieser integrierten, L-förmigen Polstern 3, 4 und einer, mindestens im Bereich der Ferse vorgesehenen Fußbettstütze 5, gebildet. Die Pelotte 2, die aus zwei, zu jeder Seite des Knöchels 6 eines Fußes 7 vorgesehenen Scheiben 8, 9 besteht, ist derart ausgeführt, daß zum einen diese Scheiben über ihren bodenseitigen Rand, d. h. deren Saum 10, miteinander verbunden und mit ihrem diesem Rand entgegengesetzten Ende über den Bereich des Knöchels in Richtung Wade 11 herausgeführt sind. Die Verbindung der beiden Scheiben 8, 9 kann dabei über Bänder oder Säume 10 am bodenseitigen Rand 22 erfolgen, wobei es zweckmäßig ist, diese Bänder unter die Fußbettstütze 5 zu führen und/oder an deren Rand zu verbinden. Bei Verbindung dieser Scheiben 8, 9 unter der Fußbettstütze 5 liegen die Säume 10 zwischen dieser und der Brandsohle 12 eines Schuhs 13, und es kann die Einlage 1 als Bestandteil des Schuhs und somit in diesem integriert als auch als separate Einlage ausgeführt sein. Die Fußbettstütze 5, die mindestens den Bereich der Ferse erfaßt, ist mindestens bis zum Vorfuß anatomisch geformt und entsprechend dem Fußverlauf korrigiert.

Das Ausführungsbeispiel gem. Fig. 1 - 3 zeigt die Einlage als eine im Schuh 13 integrierte Einheit. Bedingt durch diese Integration sind die Scheiben 8, 9 mit den an ihnen vorgesehenen Polstern 3, 4 vorzugsweise hinter dem Futter 14 des Schuhs 13 eingelegt, wobei diese Scheiben bei Ausführung des Schuhs als Sportschuh mit Schaft 27 annähernd dessen ganzen Schaft bedekken können.

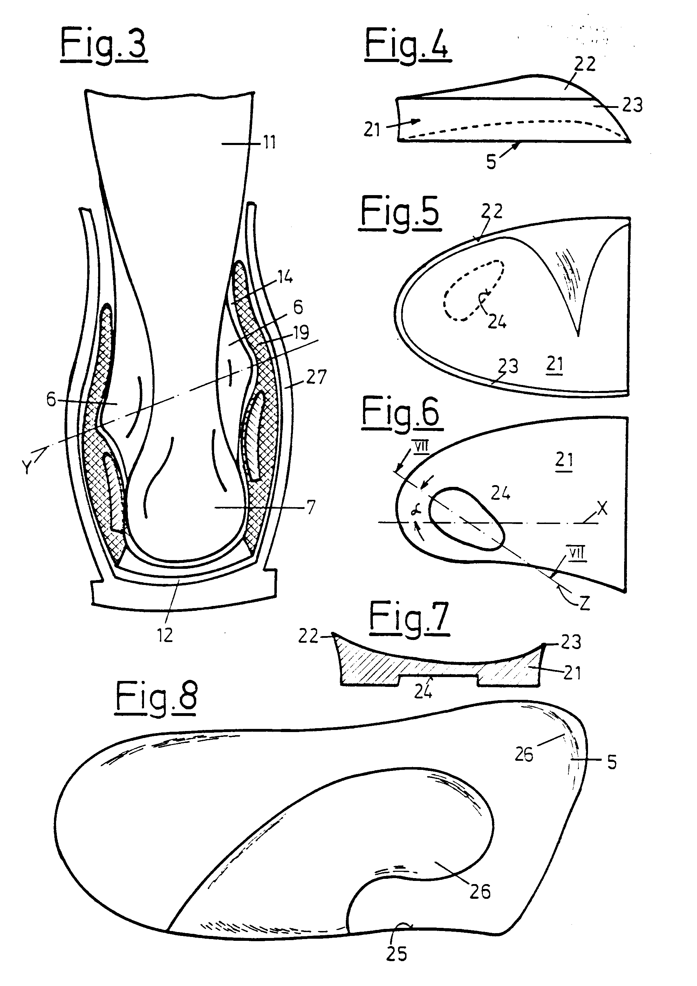

Um dabei dem Knöchel 6 die erforderliche Abstützung zu geben, sind die an den Scheiben 8, 9 angeordneten, L-förmigen Polster 3, 4 mit ihrem einen Schenkel 15 etwa parallel zur Fußbettstütze 5 und mit ihrem anderen Schenkel 16 etwa senkrecht zu dieser angeordnet. Der Eckpunkt, in dessen Bereich die Schenkel 15, 16 miteinander verbunden sind, ist sowohl innen als auch außen abgerundet, wobei die innere Abrundung 17 weich ausgeführt und deren Bogen um den überwiegenden Umfang des Knöchels 6 reicht. Diese Abrundung 17 ist wie eine Einbettung für den Knöchel 6 ausgeführt, so daß dieser, weich gedämpft, in dieser zu liegen kommt. Analog der Lage der Achse y durch den Knöchel 6, die bekanntlich nicht waagrecht verläuft, sind insb. die inneren Abrundungen 17 der L-förmigen Polster 3, 4 jeder Knöchelseite unterschiedlich hoch von der Fußbettstütze 5 angeordnet. Auch sind die Polster 3, 4 mit dem Polstermaterial soweit aufgepolstert, daß sie etwaige Hohlräume um den Knöchel 6 weitgehend ausfüllen (Fig. 3). Die L-förmigen Polster 3, 4 können von einer biegesteifen Einlage 18 und einem diese umhüllenden, weichen Polstermaterial 19, z. B. einem hautsympathischen Siliconkautschuk oder einem Schaumstoff bestehen, wobei deren Schenkel 15, 16, entsprechend der Knöchelseite, sowohl unterschiedlich lang als auch unterschiedlich stark aufgepolstert sein können. Die unterschiedliche Länge dieser Schenkel 15, 16 ist auch deshalb notwendig, als der fersenseitige und nach oben gerichtete Schenkel 16 der Führung und Abstützung der Achillessehne dient und dieser zusätzlich eine Ausbuchtung 20 haben kann, in die sich diese Achillessehne einbetten kann. Die Scheiben 8, 9, die, wie auch das Polstermaterial 19 aus dem Siliconkautschuk gefertigt sein können, fallen in Bezug auf deren Materialanhäufung von den Polstern 3, 4 zu deren Rändern 11 stark ab, so daß sie an deren Rändern eine Wandstärke von 0,5 bis 2 mm aufweisen. Um das Gewicht dieser Scheiben 8, 9 gering zu halten, kann hier statt des Siliconkautschuks auch ein anderer, ähnliche Eigenschaften aufweisender Werkstoff verwendet werden, wobei darauf zu achten wäre, daß der Werkstoff zwar dämpfend, nachgiebig ist, nach dem Dämpfungsvorgang aber in seine ursprüngliche Form zurückgeht. Solche Werkstoffe können beispielsweise Schaumstoffe sein, soweit sie formstabil ausgeführt sind.

Die Fußbettstütze 5, die je nach Art der Einlage 1 nur den Bereich der Ferse oder den der ganzen Fußsohle erfassen kann, ist auf der Brandsohle 12 des Schuhs 13 aufgelegt und dort gegebenenfalls auch mit den Bändern bzw. den Säumen 10 der Scheiben 8, 9 verbunden. Bei der Ausführung nach Fig. 4 bis 6 ist die Fußbettstütze 5 nur als sogenannte Fersenstütze 21 ausgeführt und im Schuh 13, im Bereich der Ferse, auf der Brandsohle 12 aufgelegt. Diese Fersenstütze 21 weist einen U-förmig verlaufenden Rand 22, 23, auf, wobei der dem Innenfuß zugekehrte Rand 22 höher als der dem Außenfuß zugekehrte Rand 23 sein kann. Neben diesen Rändern 22, 23 hat diese Fersenstütze 21 auch eine Ausbuchtung 24 im Bereich ihres fersenseitigen Umfanges, die insb. dem Einbetten des Fersensporns dient. Die Einbuchtung 24 ist gemäß dem hier dargestellten Beispiel oval ausgeführt, wobei deren Qualität dem Längsmittelschnitt etwa eines Eies entspricht. Untersuchungen haben gezeigt, daß es ratsam ist, wenn die Längsachse Z dieser ovalen Ausbuchtung 24 die Längsmittelachse X der Fersenstütze 21, d. h. die Sagittalachse des Fußes 7, in einem spitzen Winkel $\alpha$ schneidet.

Analog der hier gezeigten, kurzen Fußbettstütze 5 kann diese auch länger ausgeführt werden und damit von der Ferse bis annähernd zu den Zehen reichen. Eine solche Fußbettstütze 5 ist in Fig. 8 dargestellt. Diese Fußbettstütze 5, die natürlich auch die Ausbuchtung 24 aufweisen kann, ist mit einem zum Innenfuß aufsteigenden Rand 25 versehen, und es geht von diesem Rand eine Aufpolsterung 26 aus, die bis etwa zum Fußballen hin reicht. Diese Aufpolsterung 26, die vom Rand 25 bogenförmig zum Fußballen reicht, wirkt sowohl als Abstützung für die Fußsohle, als auch als Bremse für den Fuß 7, besonders dann, wenn sich dieser beim Gehen nach vorne bewegt.

Die hier gezeigte Einlage 1 bestehend aus den gepolsterten Scheiben 8, 9 und der jeweiligen, kurzen oder langen Fußbettstütze 5 ist als Bestandteil eines Schuhs 13, vorzugsweise mit Schaft 27, ausgeführt und im Schuh so integriert, daß sie hinter dem Futter 14 des Schuhs liegt. Selbstverständlich kann sie aber auch als separate Einlage 1 ausgeführt werden, die in den jeweiligen Schuh 13 eingelegt wird. Bei Ausführung derselben als separate Einlage 1 empfiehlt es sich, diese in ein elastisches Gewirk einzustricken und nach Art eines Strumpfes über den Fuß 7 zu ziehen.

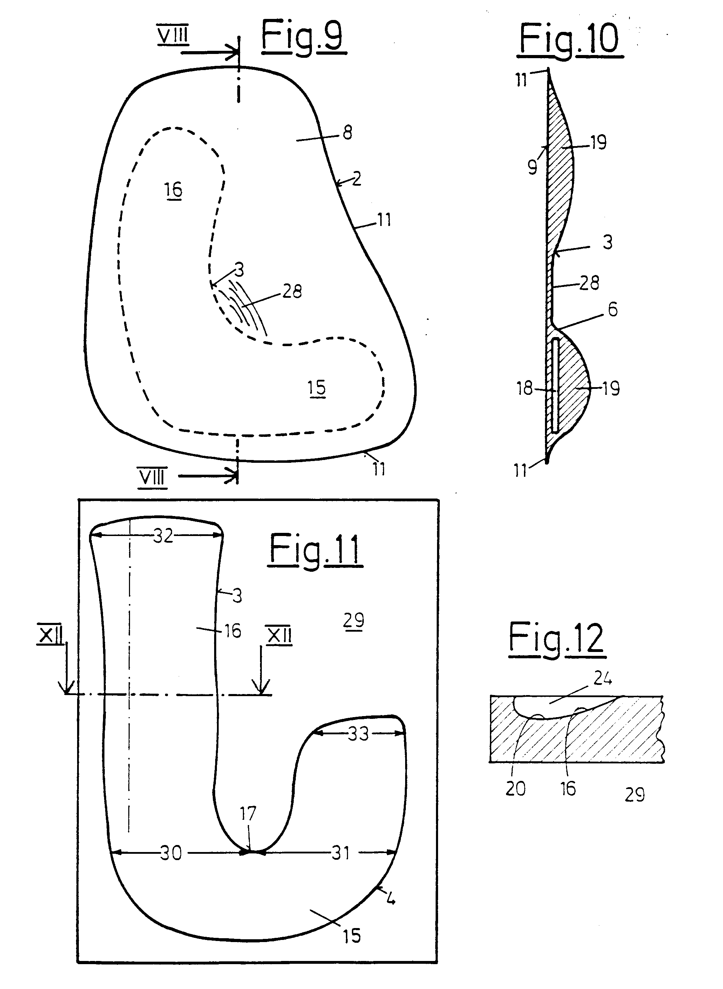

Um die Scheiben 8, 9 und die an ihnen vorgesehenen Polster 3, 4 besser zu verstehen, wird auf die Fig. 9 verwiesen. In dieser Figur ist die Scheibe 8 nebst L-förmigem Polster 3 für die innere Knöchelseite dargestellt. Die Scheibe 8 ist so groß ausgeführt, daß sie sowohl die unterschiedlichen Längen der Schenkel 15, 16 in sich aufnehmen als auch selbst in den 27 des Schuhs 13 eingelegt werden kann. Dabei kann die dem Schaft 27 zugewandte Seite der Scheibe 8 relativ glatt nach unten verlaufen, während ihre dem Knöchel 6 zugewandte Seite die Aufpolsterung 19 aufweist und demzufolge nach außen, d. h. zum Knöchel 6 hin, gewölbt ist. Die Polsterung 19 bzw. Wölbung wird dadurch dargestellt (Fig. 10), daß der Schnitt XIII-XIII durch die Pelotte 2 durch die Ausbuchtung 28 für den Knöchel 6 führt und hier den Schenkel 15 für die Abstützung dieses Knöchels 6 im Schnitt zeigt.

Es muß hier festgehalten werden, daß die L-förmigen Polster 3, 4 in den dargestellten Ausführungen zwischen ihren Schenkeln 15, 16 einen Winkel von etwa 90° schließen. Dies ist nicht zwingend erforderlich, wie auch nicht üblich, daß die Knöchel 6 aller Menschen in der gleichen Gelenkachse y liegen. Hier ist es erforderlich, den gängigsten Winkel, d. h. zwischen 80 und 110°, zu wählen und Abweichungen hiervon mit speziell gefertigten Orthesen bzw. Pelotten 2 zu bedienen.

Eine solche spezielle Pelotte 2 zeigt die Fig. 11, der die Gießform 29 für zwei L-förmige Polster 3, 4 zugrunde liegt. Die Polster 3, 4 sind für die Abstützung des äußeren Knöchels 6 gedacht, und es ist der senkrechte Schenkel 16 hier etwa doppelt so lang als der waagrechte Schenkel 15 ausgeführt. Bei dieser Darstellung ist der waagrechte Schenkel 15 ab seiner Verbindung mit dem senkrechten Schenkel 16 ebenfalls nach oben gezogen, so daß der Knöchel 6 seine Einbettung in der inneren Wölbung 17 zwischen diesen Schenkeln finden kann. Untersuchungen haben ergeben, daß es zweckmäßig ist, die Breite der Schenkel 15, 16 an ihren Wurzeln 30, 31 gegenüber deren auskragenden Enden 32, 33 im Verhältnis von 1:1,1 bzw. 1:1,3 auszuführen. Dabei sollten die freien Enden 32, 33 dieser Schenkel 15, 16 ab deren Wurzeln 30, 31 fächerartig aufgehen. Um auch die Achillessehne schützend einzubetten, sollte der ihr zugeordnete Schenkel 16 die Ausbuchtung 20 aufweisen, so daß die Sehne annähernd druckfrei in dieser Ausbuchtung ruhen kann.

Bezüglich der materialtypischen Dämpfungseigenschaft (d. h. der Viskoelastizität) sollte z. B. das Material der Fußbettstütze 5 in seinem Druckverhalten der Dämpfungsfunktion des Unterhautfettgewebes an der Fußsohle mit seiner Matratzenkonstruktion entsprechen.

**BEZUGSZEICHEN-LISTE**

| | |
|---|---|
| 1 | Einlage |
| 2 | Pelotte |
| 3 | Polster |
| 4 | Polster |
| 5 | Fußbettstütze |
| 6 | Knöchel |
| 7 | Fuß |
| 8 | Scheibe |
| 9 | Scheibe |
| 10 | Saum |
| 11 | Rand |
| 12 | Brandsohle |
| 13 | Schuh |
| 14 | Futter |
| 15 | Schenkel |
| 16 | Schenkel |
| 17 | innere Abrundung |
| 18 | Einlage (biegesteif) |
| 19 | Polstermaterial |
| 20 | Ausbuchtung |
| 21 | Fersenstütze |
| 22 | Rand |
| 23 | Rand |
| 24 | Ausbuchtung |
| 25 | Rand |
| 26 | Aufpolsterung |
| 27 | Schaft |
| 28 | Ausbuchtung |
| 29 | Gießform |
| 30 | Wurzel |
| 31 | Wurzel |
| 32 | auskragendes Ende |
| 33 | auskragendes Ende |

Sonstige

| | |
|---|---|
| y | Achse (Gelenkachse) |
| Z | Längsachse |
| x | Längsmittelachse |
| $\alpha$ | Winkel |

**Patentansprüche**

1. Medizinisch orientierte Einlage für Schuhe, insb. Sportschuhe, wie Tennisschuhe, Basketballschuhe u. a., mit über dem Knöchel des Fußes reichendem Schaft und mit seitlich des Knöchels anlegbaren und diesen mindestens teilweise umgreifenden Polstern, wobei die Polster jeder Seite des Knöchels von zwei L-förmig zueinander stehenden Schenkeln gebildet und der Knöchel durch diese einerseits vertikal und andererseits rückwärtig abstützbar ist, dadurch gekennzeichnet, daß die Polster (3, 4) an zwei lappenförmigen Pelotten (2) angebracht und diese Pelotten untereinander und über eine anatomisch geformte Fußbettstütze (5) miteinander verbunden sind, und daß diese Fußbettstütze an ihrem der Ferse des Fußes (7) zugewandten Ende eine dem Druck der Ferse nachgebende, elastische Ausbuchtung (24) aufweist, und daß diese Fußbettstütze im Bereich des Fußgewölbes einerseits eine den Sagittalschub dämpfend bremsende Aufpolsterung (26) und andererseits zwischen dieser und der Ferse eine die Außenseite des Fußes führende und stützende Auflage (22, 23) mit randseitiger Aufkrempelung aufweist.

2.  Einlage nach Anspruch 1, dadurch gekennzeichnet, daß die Pelotten (2) an ihren der Fußbettstütze (5) zugewandten Seite einen Saum (10) aufweisen, und daß diese über diese Säume sowohl miteinander als auch mit dem Schuh (13), insb. dessen Brandsohle (12), verbunden sind, und daß die Fußbettstütze über diese Säume auf dem Fußbett bzw. der Brandsohle aufgelegt ist.

3.  Einlage nach Anspruch 1, dadurch gekennzeichnet, daß die Polster (3, 4) und/oder die Fußbettstütze (5) aus einem elastischen Werkstoff (Siliconkautschuk oder Schaumstoff) hergestellt sind.

4.  Einlage nach Anspruch 1, dadurch gekennzeichnet, daß die Polster (3, 4) aus einem elastischen, jedoch inkompressiblen Kunststoff (Polyvinylchlorid) bestehen.

5.  Einlage nach Anspruch 1, dadurch gekennzeichnet, daß die Pelotten (2) als anatomisch formgerichtete Scheiben (8, 9) mit den an ihnen angebrachten, L-förmigen Polstern (3, 4) ausgeführt sind.

6.  Einlage nach Anspruch 5, dadurch gekennzeichnet, daß die Scheiben (8, 9) außerhalb des den Knöchel (6) stützenden Bereichs bzw. deren Polster (3, 4) eine Wandstärke zwischen 0,5 und 2 mm aufweisen.

7.  Einlage nach Anspruch 1, dadurch gekennzeichnet, daß die Pelotten (2) im Bereich der Abstützung des Knöchels (6) die Einfallstellen des Fußes (7) um den Knöchel ausfüllende Verstärkungen (18, 19) aufweisen.

8.  Einlage nach Anspruch 1, dadurch gekennzeichnet, daß diese, bestehend aus den Polstern (3, 4) und Pelotten (2), wie auch der Fersenstütze (21), die mindestens bis zum Vorfuß anatomisch geformt und korrigiert ist, Bestandteil des Schuhes (13) ist und diese Einlage (1) in diesem eingearbeitet ist.

9.  Einlage nach Anspruch 1, dadurch gekennzeichnet, daß die Fußbettstütze (5) nur im Bereich der Ferse des Fußes (7) im Schuh (13) eingelegt ist und deren dort vorgesehene, elastische Ausbuchtung (24) mit ihrer Öffnung dem Fußbett bzw. der Brandsohle (12) zugewandt ist.

10. Einlage nach Anspruch 9, dadurch gekennzeichnet, daß die Ausbuchtung (24) einen ovalen Grundriß aufweist, und daß die Längsachse (7) dieser Ausbuchtung die Längsmittelachse (X), d. h. die Sagittalachse des Fußes (7) in einem spitzen Winkel (α) insb. zwischen 20 und 30 Grad, schneidet.

11. Einlage nach Anspruch 9, dadurch gekennzeichnet, daß die Ausbuchtung (24) eiförmigen Grundriß aufweist und mit ihrem größeren Durchmesser im Bereich des Fersenendes und mit ihrem kleineren Durchmesser im Bereich der aufsteigenden, seitlichen Abstützung (Rand 22) der Fußbettstütze (5) liegt.

## Claims

1.  Medically-oriented supplement for shoes, typically sport shoes, such as tennis shoes, basketball shoes etc., with a shaft extending over the ankle of the foot and with cushions applicable laterally to the ankle and cushions surrounding the ankle at least partially, with the cushions on each side of the ankle being formed with legs in an L-configuration relative to each other and where the ankle is supportable both vertically and rearwardly by said legs, characterized in that the cushions (3, 4) are attached to two plate-shaped ankle supports (2) and in that said ankle supports are connected to each other via an anatomically shaped plantar support (5) and in that this plantar support is formed at its end facing the heel (7) of the foot with a cup (24), which yields flexibly to the pressure of the heel and in that this plantar support is formed in the area of the arch of the foot on the one side with padding (26) which dampens and brakes the sagittal thrust and on the other side with a bearing surface (22, 23) which guides and supports the outer side of the foot with raised borders.

**2.** Supplement as in Claim 1, characterized in that the ankle supports (2) are formed at their side facing the plantar support (5) with a seam (10) and in that said plates are connected via said seams both with each other and also with the shoe (13), typically the insole (12) of the shoe and in that the plantar support is placed via these seams on the sole or, respectively, insole.

**3.** Supplement as in Claim 1, characterized in that the cushions 3, 4 and/or the plantar support (5) are made of an elastic material (silicone rubber or foamed plastic).

**4.** Supplement as in Claim 1, characterized in that the cushions 3, 4 consist of an elastic but incompressible plastic (polyvinyl chloride).

**5.** Supplement as in Claim 1, characterized in that the ankle supports (2) are constructed as anatomically shaped plates (8, 9) with the L-shaped cushions (3, 4) attached to said plates.

**6.** Supplement as in Claim 5, characterized in that the plates (8, 9) are formed outside the area supporting the ankle (6) or the cushions (3, 4) of said plates with a wall thickness between 0.5 and 2 mm.

**7.** Supplement as in Claim 1, characterized in that the ankle supports (2) in the area where the ankle (6) is supported are formed with reinforcement (18, 19) filling the depressions of the foot (7).

**8.** Supplement as in Claim 1, characterized in that said supplement, consisting of the cushions (3, 4) and ankle supports (2) as well as the heel support (21) which is anatomically shaped and corrected at least as far as the forefoot, is an integral part of the shoe (13), and in that said supplement (1) is integrated in said shoe.

**9.** Supplement as in Claim 1, characterized in that the plantar support (5) is placed in the shoe (13) only in the area of the heel (7) of the foot and the opening of its elastic cup (24) provided there faces the plantar support or, respectively, insole (12).

**10.** Supplement as in Claim 9, characterized in that the cup (24) has an oval shape in plan and in that the longitudinal axis (7) of this cup intersects the longitudinal centre line (X), i.e.

the sagittal axis of the foot (7), at an acute angle ($\alpha$) typically between 20 and 30 degrees.

**11.** Supplement as in Claim 9, characterized in that the cup (24) is egg-shaped in plan and with its major diameter lies in the area of the heel end and with its minor diameter in the area of the rising lateral support (border 22) of the plantar support (5).

**Revendications**

**1.** Semelle orthopédique à utilisation médicale, pour chaussures, notamment pour chaussures de sport, comme par exemple les chaussures de tennis, les chaussures de basket, etc., comportant une tige dépassant la cheville du pied et des rembourrages pouvant être rapportés sur le côté de la cheville et entourant cette dernière au moins partiellement, le capiton de chaque côté de la cheville étant formé de deux branches formant entre elles un L, la cheville pouvant, par ces dernières, s'appuyer d'une part verticalement et d'autre part vers l'arrière, caractérisée en ce que les capitons (3, 4) sont rapportés à deux pelotes (2) en forme de pattes et que ces pelotes sont disposées l'une sous l'autre et sont reliées l'une à l'autre par l'intermédiaire d'un appui de semelle orthopédique (5) ayant une forme anatomique, et que cet appui de semelle orthopédique présente, en son extrémité dirigée vers le talon du pied (7), un renfoncement élastique (24), qui subit un fléchissement sous l'effet de la pression du talon, et que cet appui de semelle orthopédique comporte, dans la zone de la voûte plantaire, d'une part un capitonnage (26) qui freine avec amortissement la poussée sagittale, et d'autre part, entre ce capitonnage et le talon, une assise de revêtement (22, 23), qui soutient et guide le côté extérieur du pied et présente un retroussement sur les bords.

**2.** Semelle orthopédique selon la revendication 1, caractérisée en ce que les pelotes (2) comportent un ourlet (10) sur leur côté dirigé vers l'appui de semelle orthopédique (5), et que ces pelotes sont, par l'intermédiaire de cet ourlet, reliées tant l'une à l'autre qu'à la chaussure (13), en particulier sa semelle intérieure (12), et que l'appui de semelle orthopédique est disposé, par l'intermédiaire de ces ourlets, sur la semelle orthopédique ou la semelle intérieure.

**3.** Semelle orthopédique selon la revendication 1, caractérisée en ce que les capitons (3, 4) et/ou les appuis de semelle orthopédique (5) sont réalisés en un matériau élastique (caoutchouc siliconé ou matériau expansé).

4.  Semelle orthopédique selon la revendication 1, caractérisée en ce que les capitons (3, 4) sont en un matériau plastique élastique mais incompressible (polychlorure de vinyle)).

5.  Semelle orthopédique selon la revendication 1, caractérisée en ce que les pelotes (2) sont réalisées sous forme de disques (8, 9), de forme anatomique, avec les capitons (3, 4) en forme de L qui y sont rapportés.

6.  Semelle orthopédique selon la revendication 5, caractérisée en ce que les disques (8, 9) présentent, à l'extérieur de la zone qui soutient la cheville (6), ou à l'extérieur de ses capitons (3, 4), une épaisseur de paroi de 0,5 à 2 mm.

7.  Semelle orthopédique selon la revendication 1, caractérisée en ce que les pelotes comportent, dans la zone de l'appui de la cheville (6), des renforcements (18, 19) qui remplissent, autour de la cheville, les points de contact du pied (7).

8.  Semelle orthopédique selon la revendication 1, caractérisée en ce que cette semelle orthopédique, constituée des capitons (3, 4) et des pelotes (2), de même que de l'appui de talon (21), qui au moins jusqu'à l'avant-pied a une forme anatomique et est corrigé en conséquence, fait partie intégrante de la chaussure (13), cette semelle orthopédique (1) y étant incorporée.

9.  Semelle orthopédique selon la revendication 1, caractérisée en ce que l'appui de semelle orthopédique (5) n'est inséré dans la chaussure (13) que dans la zone du talon du pied (7), son renfoncement élastique (24), qui y est prévu, étant par son ouverture dirigé vers la semelle orthopédique ou la semelle intérieure (12).

10. Semelle orthopédique selon la revendication 9, caractérisée en ce que le renfoncement (24) a une forme ovale, et que l'axe longitudinal (7) de ce renfoncement coupe l'axe longitudinal central (X), c'est-à-dire l'axe sagittal du pied (7), selon un angle aigu ($\alpha$), en particulier compris entre 20 et 30 degrés.

11. Semelle orthopédique selon la revendication 9, caractérisée en ce que le renfoncement (24) a une forme ovale et, par son grand diamètre, se trouve dans la zone de l'extrémité du talon et, par son petit diamètre, se trouve dans la zone de l'appui latéral montant (bord 22) de l'appui de semelle orthopédique (5).

Fig.1

Fig.2

# Fig.3

11

14
6
19
27
6
7
Y
12

# Fig.4

22
21
23
5

# Fig.5

22
24
23
21

# Fig.6

VII
21
24
X
VII
Z

# Fig.7

22
23
21
24

# Fig.8

26
5
26
25

# Fig.9

VIII

VIII

8

2

16

11

3

28

15

11

# Fig.10

11

19

9

3

28

6

18

19

11

# Fig.11

32

3

16

XII

XII

29

33

17

30

31

4

15

# Fig.12

24

20

16

29